# EUROPEAN PATENT APPLICATION

(11) **EP 3 476 409 A1**
(43) Date of publication of application: **01.05.2019**
(21) Application number: 18159541.4
(22) Date of filing: 01.03.2018
(51) Int. Cl.: A61L 27/26, A61L 27/48, A61L 27/52

(54) **COMPOSITE HYDROGEL FOR CELL ENCAPSULATION COMPRISING COLLAGEN AND SILK FIBROIN, AND PREPARATION METHOD THEREOF**

(30) Priority: 30.10.2017 KR 20170142691
(71) Applicant: Dankook University Cheonan Campus Industry Academic Corporation Foundation, Cheonan-si, Chungcheongnam-do 31116 (KR)
(72) Inventor: KIM, Hae-Won, 31170 Cheonan-si, Chungcheongnam-do (KR); LEE, Jung Hwan, 05217 Gangdong-gu, Seoul (KR); BUITRAGO, Jennifer O., 31116 Cheonan-si, Chungcheongnam-do (KR); CAMPBELL KNOWLES, Jonathan, St Albans, Hertfordshire AL3 5AJ (GB)
(74) Representative: Regimbeau

(57) **Abstract**

The present invention relates to a method for preparing a composite hydrogel for cell encapsulation comprising collagen and silk fibroin, comprising forming a gel by mixing collagen having an α-helix structure and silk fibroin having a β-sheet structure; and to a composite hydrogel for cell encapsulation prepared accordingly. The composite hydrogel of the present invention exhibits stiffness in the range of 0.05 kPa to 40 kPa, which is suitable for most tissues; allows cell encapsulation; assists cell adhesion and proliferation; restrains shrinkage exerted by cell traction forces; and shows a beneficial effect for long-term *in vitro* cultures and *in vivo* tissue engineering.

## Description

### [Invention Title]

Composite hydrogel for cell encapsulation comprising collagen and silk fibroin, and preparation method thereof

### [Technical Field]

The present invention relates to a composite hydrogel and a preparation method thereof. Specifically, the present invention relates to a composite hydrogel composed of collagen and silk fibroin, which has excellent physical, mechanical, and biological properties, and a preparation method thereof.

### [Background Art]

Tissue engineering is a multi-disciplinary, translational science aimed at developing clinically effective substitutes for treating damaged and diseased tissues. The design of biocompatible materials having proper viscoelastic properties consistent with a naturally occurring niche of stem cells is one of the major strategies for regeneration of damaged tissues.

Tissues have a different composition and structure of extracellular matrix (ECM), and the resultant mechanical properties (*e.g*., stiffness) that can direct stem cells to proliferate and differentiate appropriately to lineage cells. For instance, soft tissues (*e.g*., brain) have a stiffness around 0.1 kPa to 1 kPa; cells in slightly stiffer tissues such as muscle prefer 5 kPa to 10 kPa; and the stiffness of collagenous bone matrix is over 25 kPa. Cells are able to detect the external mechanical forces triggered in the ECM adhesive proteins (laminin, collagen, and fibronectin) which are physiologically linked to cytoskeletal actin filaments through adhesion complexes, mainly integrins, located in the cell membrane. The cytoskeleton is also physically coupled to a nucleus through proteins at the nuclear envelope, and thus the ECM signals can transmit physically through cytoskeletal networks toward nucleus molecules, and can then be translated into biochemical signals to ultimately upregulate/downregulate cellular responses including proliferation, migration, and/or differentiation.

Hydrogels are considered to be optimal for providing physicochemical signals to cells in a manner similar to natural tissues. Hydrogels are hydrophilic polymers cross-linked by a cohesive force such as a covalent bond, a hydrogen bond, a van der Waals bond, or a physical bond; and are materials having a three-dimensional polymer network structure capable of being swelled because a large amount of water is contained therein in an aqueous solution. If cell-containing hydrogels are injectable, these are suitable for minimally invasive surgery because of the potential thereof for medical applications. Various synthetic materials such as poly(ethylene oxide) (PEO), poly(vinyl alcohol) (PVA), and poly(acrylic acid) (PAA), and various natural materials such as agarose, alginate, chitosan, collagen, fibrin, gelatin, and hyaluronic acid (HA) have been used to form hydrogels. Gelation occurs when polymer chains are induced by a chemical agent (*e.g.,* a cross-linking agent) or a physical stimulus (*e.g.,* pH and/or temperature), followed by forming a network structure because the induced polymers chains are chemically and physically cross-linked. In general, natural polymers are more closely related to extracellular matrices and have polymeric properties whereby decomposition products are non-toxic. Therefore, the natural polymers tend to be useful as carriers for cell and bioactive molecules for tissue engineering and implantable medical devices, but the gelation thereof is less controllable.

Recently, silk and collagen, two typical important biological proteins, have been used as hydrogel materials. Collagen is the most abundant protein component in the ECM of various tissues such as skin, bones, tendons, ligaments, muscles, and blood vessels. In fact, many attempts have been made to produce collagen hydrogels with various stiffness values by changing the concentration. Although shear modulus (G') of collagen hydrogels was observed to be concentration-dependent (2.2 Pa at 0.4 mg/mL, 7 Pa at 1 mg/mL, 14 Pa at 1.5 mg/mL, 15 Pa to 17 Pa at 2 mg/mL, and 120 Pa at 4 mg/mL), the obtained gels are considered to have very low stiffness levels (mostly lower than 1 kPa) without chemical cross-linking. Additionally, the collagen gels have limitations that cannot be applied to cell encapsulation and delivery when cross-linked.

On the other hand, silk fibroin proteins, which have received attention for the past two decades, are regarded as promising natural polymers due to their good biocompatibility and high mechanical properties. Rheological measurement of silk fibroin hydrogels revealed G' values in a wide kPa range (4.8 kPa at 1%, 7.4 kPa at 2%, 22.4 kPa at 3%, 33.1 kPa at 4%, and 70 kPa at 5.2%); that is, the G' values are much higher than those obtained in collagen hydrogels. However, the silk concentrations of less than 4% are difficult to handle, and the stiffness level implemented is too high for optimal application to many soft tissues such as nerves. Additionally, *Bombyx mori,* the most widely known silk fibroin, lacks RGD sequences, limiting potential cell adhesion and growth.

The present inventors have acknowledged the problem above and studied a composite hydrogel composed of collagen and silk fibroin which has excellent physical, mechanical, and biological properties. As a result, the present inventors have found that when collagen having an α-helix structure and silk fibroin having a β-sheet structure are mixed, gelation is accelerated; a composite hydrogel having adjustable stiffness can be prepared because a network is formed by molecular interaction between the collagen and silk fibroin proteins; and have also found that the prepared composite hydrogel can be used for cell encapsulation, thereby completing the present invention.

### [Prior-Art Document]

### [Patent Document]

(Patent Document 1) KR10-2015-091421 A

### [Disclosure]

### [Technical Problem]

An object of the present invention is to provide an optimal hydrogel for cell delivery and target tissue engineering, and a preparation method thereof.

Another object of the present invention is to provide a composite hydrogel composed of collagen and silk fibroin having excellent physical, mechanical, and biological properties, and a preparation method thereof using a non-chemical, cell-friendly method.

In the present invention, the hydrogel matrix having adjustable stiffness may provide appropriate biophysical/chemical signals to the cells of the target tissue.

### [Technical Solution]

A first aspect of the present invention is to provide a method for preparing a composite hydrogel for cell encapsulation comprising collagen and silk fibroin, which comprises forming a gel by mixing collagen having an α-helix structure and silk fibroin having a β-sheet structure.

A second aspect of the present invention is to provide a composite hydrogel for cell encapsulation comprising collagen and silk fibroin, wherein: the composite hydrogel comprises collagen having an α-helix structure and silk fibroin having a β-sheet structu re, in which an interpenetrating network between the collagen having an α-helix structure and the collagen having a β-sheet structure is formed by a hydrogen bond; and the stiffness of the composite hydrogel is adjusted within the range of 0.05 kPa to 40 kPa.

Hereinbelow, the present invention will be described in detail.

Hydrogels behave as viscoelastic materials with an elastic component determined by the network of cross-linked polymers, and a viscous component including a high content of water in their composition. The hydrogels should be able to satisfy good biocompatibility, allow cell encapsulation, and further aid cell growth and function. In addition, the hydrogels should have a broad range of adjustable stiffness such that they can direct stem cells to multiply in a different composition and structure of extracellular matrix (ECM) and to differentiate appropriately to lineage cells. Conventional collagen gels have very low stiffness levels without chemical cross-linking, and have limitations that cannot be applied to cell encapsulation and delivery when cross-linked. In addition, since silk concentrations of less than 4% are difficult to handle and the stiffness level implemented is too high, silk fibroin gels cannot be optimally applied to many soft tissues such as nerves.

Under these circumstances, the present inventors have studied a composite hydrogel composed of collagen and silk fibroin, which has excellent physical, mechanical, and biological properties. As a result, they have found that when collagen (0.5 mg/mL to 4 mg/mL) having an α-helix structure and silk fibroin (0.35% w/v to 2.45% w/v) having a β-sheet structure are mixed, gelation is accelerated; a composite hydrogel having adjustable stiffness in the range of 0.05 kPa to 40 kPa can be prepared because a network is formed by molecular interaction between the collagen and silk fibroin proteins; and that the prepared composite hydrogel can be used for cell encapsulation. The present invention is based on these findings.

The preparation method of the present invention is characterized in that gelation is accelerated by mixing collagen having an α-helix structure and silk fibroin having a β-sheet structure, and that an interpenetrating network is formed by molecular interaction between the collagen with an α-helix structure and the silk fibroin with a β-sheet structure.

To this end, the method of the present invention for preparing a composite hydrogel comprises a step of neutralizing the collagen to be self-assembled into collagen having an α-helix structure by neutralization. Collagen has a twisted triple-helix structure wrapped around a left-circular helix where three polypeptide chains are wrapped to the left. The collagen having a triple-helix structure cannot form a gel by mixing with silk fibroin having a β-sheet structure, and also cannot form a network by molecular interaction. Therefore, in the present invention, the pH of the collagen having a triple-helix structure is neutralized with NaOH, *etc.* so that the collagen is self-assembled into that having an α-helix structure.

Additionally, in the preparation method of the present invention, the silk fibroin is prepared by ultrasonicating the silk fibroin to be self-assembled into silk fibroin having a β-sheet structure.

The silk fibroin may be one from which sericin is removed.

When fibrous collagen with an α-helix structure and silk fibroin with a β-sheet structure, the structures of which are different, are mixed, gelation progresses by forming an interpenetrating network.

After ultrasonication, silk fibroin undergoes a rapid structural change from random coils to β-sheets (X. Wang et al., Biomaterials. 29 (2008)). In an embodiment of the present invention, it was confirmed that the peak of the silk fibroin was observed only at 1638 cm⁻¹ (no peak at 1620 cm⁻¹) without ultrasonication. However, as a result of the ultrasonication at 20% amplitude for 10 seconds or more, a strong peak of a β-sheet structure was observed. Therefore, the ultrasonication time is preferably 10 seconds or more at 20% amplitude, more preferably 10 seconds to 60 seconds.

The ultrasonication is known in the art. For such applications, the terms "ultrasonication" and "sonication" are used interchangeably and have the same definition. The ultrasonication may be performed in any manner known in the art that applies ultrasonic waves to silk fibroin. The ultrasonication may be conducted by exposing silk fibroin once to sonication, or by accompanying multiple separate exposures.

In the present invention, it was confirmed that gelation stably proceeded when the concentration of the collagen having an α-helix structure and that of the silk fibroin range from 0.5 mg/mL to 4 mg/mL, and 0.35% w/v to 2.45% w/v, respectively.

Collagen promotes the gelation of silk fibroin, and such effect is especially noticeable when the silk concentration is low. It was confirmed in an embodiment of the present invention that when the silk with the concentration of 0.3% to 0.5% is not mixed with collagen, gels could not be formed by ultrasonication; and that the mixing of the silk and collagen significantly accelerated the gelation. Such results suggest that molecular interaction between silk and collagen proteins may occur.

An interpenetrating network (IPN) may be formed between the α-helical collagen and β-sheet silk fibroin by hydrogen bonding of the amino acid residues of these two proteins. The interpenetrating network refers to two or more networks that are partly staggered in molecular units, rather than formed by covalent bonds. Both silk and collagen can form this interpenetrating network (IPN); both are interlaced, and although covalent bonds are not involved, both phases can be separated if chemical bonds are broken, and thus the IPN polymers readily show improved physical and mechanical properties.

The preparation method of the present invention is characterized in that the stiffness is adjusted within a range of 0.05 kPa to 40 kPa by adjusting the concentrations of the collagen and silk fibroin. Accordingly, the composite hydrogel of the present invention may have the stiffness ranging from 0.05 kPa to 40 kPa, which satisfies various stiffness values suitable for most soft and hard tissues.

The mechanical and biological properties of the composite hydrogel of the present invention can be adjusted to represent the combined advantages of individual proteins. In particular, the shear storage modulus may be adjusted to range from 0.05 kPa to 40 kPa along an iso-compositional line. Therefore, the composite hydrogel of the present invention may be suitable for cells of soft tissues (*e.g*., brain) having a stiffness of about 0.1 kPa to 1 kPa, cells of muscle tissues having a stiffness of about 5 kPa to 10 kPa, and cells of collagen bones having a stiffness of 25 kPa or higher.

Specifically, the composite hydrogel of the present invention may be one that has been adjusted to have a stiffness of 0.1 kPa to 1 kPa for application to cells of soft tissues (*e.g*., brain).

Additionally, the composite hydrogel of the present invention may be one that has been adjusted to have stiffness of 5 kPa to 10 kPa for application to cells of muscle tissues.

Additionally, the composite hydrogel of the present invention may be one that has been adjusted to have stiffness of 25 kPa or higher for application to collagen bones.

Specifically, the present invention provides a preparation method of a composite hydrogel for cell encapsulation comprising collagen and silk fibroin, which comprises: neutralizing the collagen to be self-assembled into collagen having an α-helix structure; ultrasonicating the silk fibroin to be self-assembled into silk fibroin having a β-sheet structure; forming a gel by mixing the collagen having an α-helix structure (0.5 mg/mL to 4 mg/mL) and the silk fibroin having a β-sheet structure (0.35% w/v to 2.45% w/v); and forming an interpenetrating network by molecular interaction, wherein the stiffness of the hydrogel is adjusted in the range of 0.05 kPa to 40 kPa by adjustment of the concentrations of the collagen and silk fibroin.

The composite hydrogel of the present invention exhibited an excellent elastic behavior under various stress modes, including bending, twisting, stretching, and compression, which was rarely observed in pure collagen or silk hydrogel.

The composite hydrogel of the present invention allows cell encapsulation; assists cell adhesion and proliferation; restrains shrinkage exerted by cell traction forces; and shows a long-term beneficial effect with respect to *in vitro* cultures and *in vivo* tissue engineering.

The collagen component in the composite hydrogel of the present invention provides adhesive sites to cells, stimulating anchorage and spreading significantly with respect to silk gel, which lacks cell adhesion motifs. In an embodiment of the present invention, it was confirmed that the cells (hMSC) cultured on the composite hydrogel of the present invention rapidly anchored and spread, and actively proliferated, and also did not show hydrogel shrinkage, overcoming the general limitation of both pure silk (lack of cell adhesion motifs) and pure collagen (substantial cell-mediated shrinkage).

The cells may be encapsulated within the composite hydrogel of the present invention while maintaining viability and growth under long-term 3D culture. In an embodiment of the present invention, it was confirmed that the cells could be safely encapsulated within the composite hydrogel, and that the cells exhibited high viability under long-term 3D culture. Such properties of the silk-collagen hydrogel, *i.e.,* accelerated gelation, improved physical/mechanical properties, and favorable cellular responses, result from the synergism of individuals placed in two different protein regimes.

In the composite hydrogel of the present invention, the inserted α-helical collagen fibrils and β-sheet silk structure may compensate for the weak points of individuals against an applied stress, *i.e.,* α-helical fibrils provide a high flexibility to the rigid β-sheet units while β-sheets strengthen α-helical fibrils to resist yield and water loss.

The composite hydrogel of the present invention is useful as a gel for elastic cell encapsulation, which has a wide stiffness spectrum which can be adjusted to target tissues.

### [Advantageous Effects]

The composite hydrogel of the present invention exhibits stiffness of 0.05 kPa to 40 kPa, which is suitable for most tissues, and may exhibit rapid gelation and elastic behavior properties that are differentiated from an individual hydrogel of collagen or silk.

The composite hydrogel of the present invention allows cell encapsulation, assists cell adhesion and proliferation, restrains shrinkage exerted by cell traction forces, and shows a long-term beneficial effect with respect to *in vitro* cultures and *in vivo* tissue engineering.

### [Brief Description of Drawings]

Fig. 1 is a schematic illustration showing a typical gelation process of silk-collagen composite hydrogels and a composition map of the prepared hydrogels (some representative iso-compositional lines 1:2.5, 1:3.5, 1:5, 1:7, and 1:10 indicated).

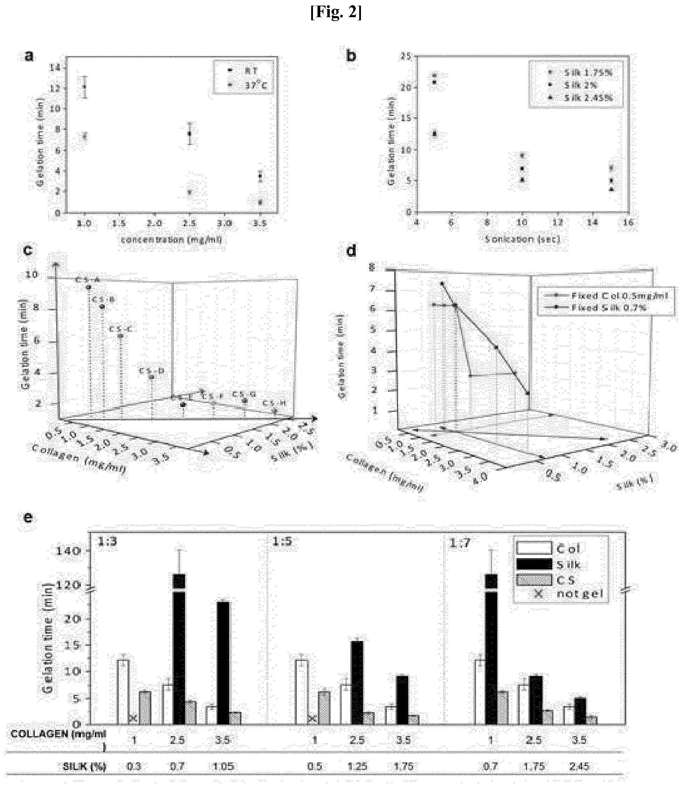

Fig. 2 is a result of the gelation time analysis of hydrogels: (a) Concentration- and temperature-dependent gelation time of pure collagen gels; (b) Ultrasonication exposure time- and concentration-dependent gelation time of pure silk gels; (c) Gelation time of collagen-silk composite gels prepared along a representative iso-compositional line (1:7); (d) Gelation time changes along the varying composition of one component (silk or collagen) at a fixed composition of the component (collagen or silk). Silk varies along red line and collagen along blue line; (e) Comparison of gelation time of collagen-silk composite gel and collagen or silk individual gel.
Fig. 3 shows shear modulus (G') plot of collagen-silk hydrogels along the iso-compositional line (*i.e*., modulus covers a wide range (0.017 kPa to 6.81 kPa)).
Fig. 4 shows mechanical behaviors of collagen-silk composite hydrogels: (a) Rectangular form of hydrogels (collagen 3.5 mg/mL, silk 2%, and CS-F as a representative group), manually subjected to different stress conditions after unmolding. CS-F maintained integrity and water content, and sustained bending and tension forces; (b) Hydrogels in a hollow tubular form after extraction are bent, twisted, and stretched to show clear difference in their behaviors.
Fig. 5 shows mechanical behaviors of collagen-silk composite hydrogels: (a) A small load applied to a cylindrical hydrogel. Collagen hydrogels significantly lost water, and thus cannot maintain the shape, whereas in pure silk and collagen-silk hydrogels, water content is well maintained, and thus the shape thereof is recovered; (b) When applying continuous forces, silk hydrogels lose integrity, whereas composite hydrogels show an excellent elasticity.
Figs. 6a and 6b show results of quantification of adherent cells on different hydrogels at 4 hours, and cell morphology; and Figs. 6c and 6d shows change in cell morphology and cell surface area at different time intervals during the first 3 hours (p < 0.05).
Fig. 7a shows cell growth morphology; Fig. 7b shows the cell number quantified during culture for 3 days on different hydrogels; and Fig. 7c shows shrinkage as a result of cell traction forces observed in collagen hydrogels but not in silk-based hydrogels (hydrogel diameter = 10 mm).
Fig. 8 shows a hydrogel, in which cells were cultured for up to 14 days, stained with live/dead cell kit, and visualized under a fluorescence microscope. Most cells alive within all composite hydrogels.
Fig. 9 shows microstructure of collagen, silk and composite hydrogels by SEM.

### [Detailed Description of the Embodiment]

Hereinbelow, the present invention will be described in detail with accompanying exemplary embodiments. However, the exemplary embodiments disclosed herein are only for illustrative purposes and should not be construed as limiting the scope of the present invention.

### Preparation of silk fibroin purified solution

Extraction of silk fibroin was performed according to a protocol previously reported in the literature (D.N. Rockwood et al., Nat. Protoc. 6 (2011) 16121631). Briefly, silk cocoons (5 g) from *Bombyx mori* (Uljinsilk, South Korea) were cut into pieces (1 cm to 1.5 cm) and then boiled for 30 minutes in 2 L of a 0.02 M sodium carbonate (Na₂CO₃, Sigma, 57795) aqueous solution to remove sericin. Thereafter, degummed cocoons were washed twice thoroughly with ultra-pure water for 20 minutes to completely wash the sericin from silk fibroin.

The produced fibroin was weighed, and then dissolved in 9.3 M lithium bromide (LiBr, Daejun, 5108-1410) to a final concentration of 20% w/v for 4 hours at 60°C. The viscous solution was then dialyzed against ultrapure water in a 3.5K MWCO dialysis membrane (ThermoFisher, 88244) for at least 48 hours to remove all salts.

The fibroin solution was centrifuged twice at 10.000 g for 20 minutes at 4°C to remove non-dissolved fibers and other impurities, and finally stored at at 4°C for later use. Fibroin was then sterilized using high-pressure steam (autoclave) at 121°C for 15 minutes. The final concentration of silk fibroin was determined to be about 6.5% w/v to 7% w/v. Desired concentrations were obtained by dilution with sterilized water.

### Preparations of rat tail-derived collagen type I and collagen hydrogels

A collagen solution (type I from a rat tail, high concentration (about 9 mg/mL to 10 mg/mL), Corning, 354249) was used. For the preparation of collagen hydrogels, DMEM 20X (Gibco, 12800-017), 0.02 N acetic acid, and 1 N NaOH were used according to the procedures reported elsewhere.

### Example: Preparation of hydrogels mixed with collagen and silk

The composition of a series of different collagen-silk hydrogels was prepared according to Table 1 below.

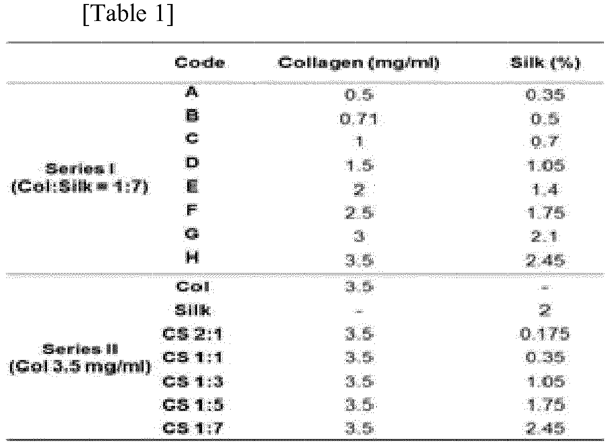

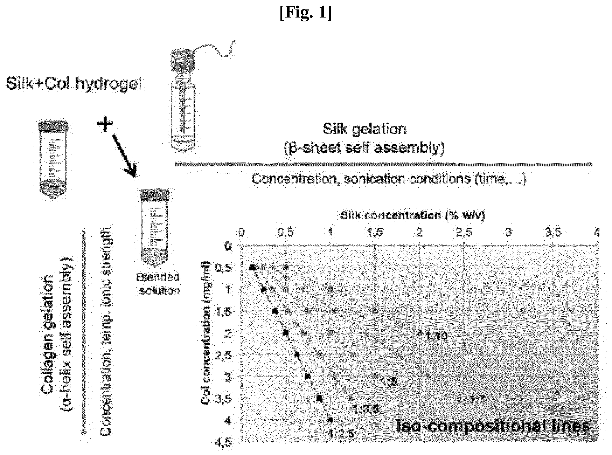

In one series, the ratio of collagen and silk was fixed at 1:7 (one of the iso-compositional lines drawn in Fig. 1); the concentrations of both proteins changed gradually while maintaining the ratio at 1:7, which results in a gradual change in stiffness at a fixed composition. The obtained hydrogels were coded from 'CS-A' to 'CS-H'. In the other series, the collagen concentration was fixed at 3.5 mg/mL, and the silk concentration was varied to 0.175%, 0.35%, 1.05%, 1.75%, and 2.45%, and therefore, different collagen/silk ratios (collagen:silk = 2:1, 1:1, 1:3, 1:5, and 1:7, respectively, and coded as 'CS 2:1' to 'CS 1:7') were obtained.

In a typical example of preparing 'CS 1:1', collagen (0.5 mL) was prepared with 0.02 N acetic acid and DMEM 20X (0.5 mL), neutralized with 0.1 M NaOH, and then placed on ice. Separately, the silk fibroin solution (1 mL) was placed in an ultrasound device (Korea Process Technology Co. Ltd., KSS-750DT), and then ultrasonicated at 20% of amplitude for 10 seconds to form β-sheets. Thereafter, the ultrasonicated silk solution (0.5 mL) was added to the collagen solution and mixed homogeneously. Particular attention was paid to avoid bubble formation during the process. The mixed solution was aliquoted to a PDMS (polydimethylsiloxane) mold, and incubated at 37°C in a humidified atmosphere to prepare hydrogel samples.

### Experimental Example 1: Gelation Test

The gelation time of the hydrogels was determined at room temperature by a vial-tilting method. 1 mL of each hydrogel solution (CS-A to H, see Table 1) was newly prepared (in triplicate), mixed, and then rapidly transferred to a glass vial (2 mL) to measure the gelation time when inverted.

**[Table 2] Other composition of collagen-silk hydrogels for observation of gelation potential**

| | Neutralization | Ultrasonication | Gelation | Observation |
|---|---|---|---|---|
| Collagen | × | | × | Liquid form; color transition from transparent to white |
| Silk | | × | | |
| Collagen | ○ | | ○ | Unstable and weak hydrogel |
| Silk | | × | | |
| Collagen | × | | × | Silk precipitation of silk and phase separation; unstable |
| Silk | | ○ | | |
| Collagen | ○ | | ○ | Stable hydrogel |
| Silk | | ○ | | |

The gelation time of pure collagen hydrogels (1 mg/mL, 2.5 mg/mL, and 3.5 mg/mL) was determined at different temperatures (4°C and 37°C). In addition, the gelation time of pure silk fibroin hydrogels was tested at various concentrations (1.75%, 2%, and 2.45%) and different ultrasonication times (5 seconds, 10 seconds, and 15 seconds).

When collagen and silk were not subjected to any treatments (neutralization at pH 7.0 for collagen and ultrasonication for silk), the mixed solution could not become a gel, but only exhibited a color change from transparent to white due to the acidic pH of collagen, which may precipitate silk fibroin protein. When collagen was neutralized but silk was not ultrasonicated, a very unstable and weak gel was produced. On the other hand, when silk was ultrasonicated but collagen was not neutralized, the low pH of the mixed solution made silk coagulated without gelation. The mixed solution became a gel only when both the collagen neutralization and the silk ultrasonication were applied.

Thereafter, the gelation time was measured over a wide compositional range. As shown in Fig. 1, the present inventors focused on the iso-compositional lines (both collagen and silk concentrations changed regularly along the fixed silk/collagen rations, *i.e*., silk:collagen = 1:10, 1:7, 1:5, 1,3.5, and 1:2.5). First, the collagen concentrations that allow gelation were observed to be limited in the range of 0.5 mg/mL to 4 mg/mL. Likewise, the limit of the silk concentrations was established at 0.35% to 2.45%.

The collagen gelation measurement was performed depending on the concentration, temperature, and ionic strength (Fig. 1). As shown in Fig. 2a, the collagen gelation time for the concentrations (1 mg/mL, 2.5 mg/mL, and 3.5 mg/mL) and temperatures (room temperature and 37°C) was plotted. With collagen (1 mg/mL), the gelation time at room temperature (about 12 minutes) decreased at 37°C (about 7.5 minutes). In addition, the gelation time decreased as the concentration of collagen increased; the times were 7.5 minutes and 3.5 minutes at 2.5 mg/mL and 3.5 mg/mL, respectively.

Similarly, the gelation of pure silk was examined by varying the ultrasonication time and silk concentration (Fig. 2b). The gelation time decreased significantly with increasing ultrasonication time (*e.g*., gelation time changed from 27 minutes to 7 minutes as ultrasonication time increased from 5 seconds to 15 seconds). In addition, the gelation time decreased as a result of increasing the silk concentration from 1.75% to 2.45%.

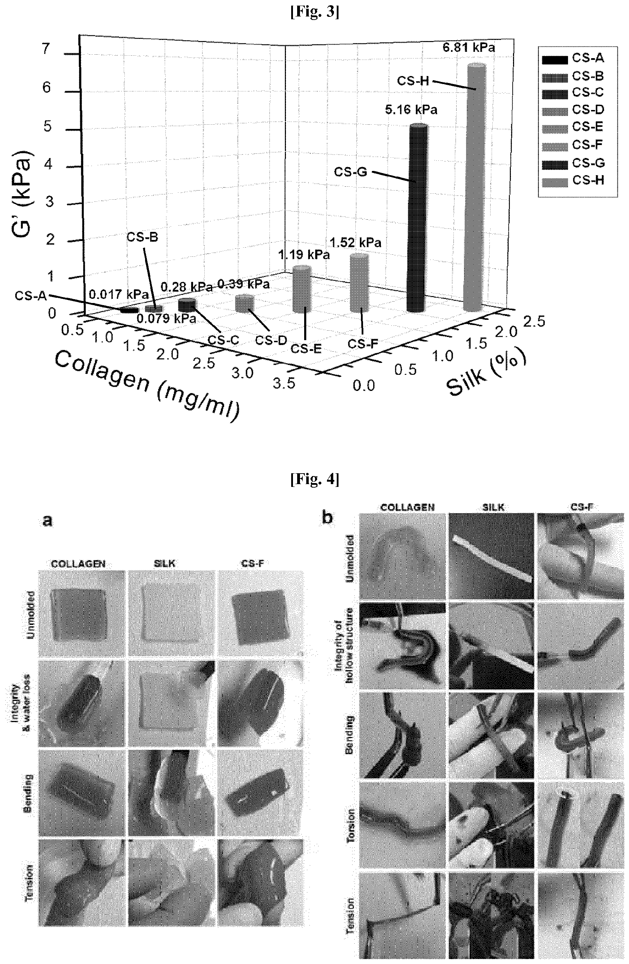

Based on the gelation information on pure collagen and silk solutions, the gelation time of the collagen-silk mixture was examined. Fig. 3c shows the change in the gelation time along the iso-compositional line (CS 1:7). At relatively low concentrations of both collagen and silk ('CS-A' to 'CS-D'), the gelation time decreased almost linearly (from 9.4 minutes to 6.26 minutes) with increasing concentration, reflecting the concentration effects of both silk and collagen; it was similarly observed in pure collagen or silk gel. At much higher concentrations ('CS-E' to 'CS-H'), the decrease in the gelation time appeared to almost saturate to about 2 minutes.

In another experiment, the gelation time was measured at a fixed concentration of either one of the two (collagen or silk) while varying the concentration of other components, as shown in Fig. 2d. At a fixed concentration of 0.75%, the increase in collagen concentration gradually reduced the gelation time. On the other hand, when the silk concentration exceeded 1% at a fixed collagen concentration of 1 mg/mL, the gelation abruptly decreased.

To better understand the gelation rates and the role for each component, different collagen/silk ratios (CS 1:3 and CS 1:5) were studied and compared in Fig. 2e. Compared with pure silk (white bars) or collagen (black bars), the mixture thereof (hashed bars) showed substantially reduced gelation time for all compositions. The effect of collagen on accelerating gelation of silk was notable, particularly when the silk concentration was low (less than 0.7% at all ratios). In fact, at CS 1:7, the gelation of 0.7% silk took about 2 hours; however, when collagen (1 mg/mL) was mixed, the gelation time decreased rapidly to 6.5 minutes (CS 1:7); and when collagen (2.5 mg/mL) was mixed, the gelation time decreased to 4.5 minutes (CS 1:3). Additionally, the silk concentrations of 0.3% to 0.5% could not form a gel by ultrasonication unless these were mixed with collagen. At CS 1:3, the mixing with collagen (1 mg/mL) successfully induced gelation within about 7 minutes. That is, the mixing of silk and collagen significantly accelerated the gelation process, suggesting that there may be molecular interactions between the silk and collagen proteins.

### Experimental Example 2: Analysis of hydrogels according to Fourier-transform infrared spectroscopy (FT-IR)

Attenuated total reflectance FT-IR (ATR-FTIR) spectroscopy was used to detect the chemical bonding status of the composite hydrogels. Slow scanning spectra (100 scans/spectrum) were collected with a resolution of 4 cm⁻¹ on an FT-IR spectrometer (Varian 640-IR, Australia) in the range of 2000 cm⁻¹ to 400 cm⁻¹ using the Gladi ATR diamond crystal accessory (PIKE Technologies, USA).

The samples used for the analysis were prepared as follows: a volume of 200 µL of each hydrogel was placed in a 96-well plate and gelated. Thereafter, the samples were washed with deionized water to completely remove excess salts and media, and then frozen at -80°C and lyophilized prior to analysis. The compositions of the analyzed samples are shown in Table 1.

The change in the chemical bond structure, which is a consequence of the possible interactions between collagen and silk, was examined by FT-IR analysis. As an *a priori* test, 2% pure silk was observed by applying different ultrasonication times to check the onset of β-sheet structure formation.

Three amide bands (amide I at 1700 cm⁻¹ to 1600 cm⁻¹, and amide II at 1600 cm⁻¹ to 1500 cm⁻¹, and amide III at 1350 cm⁻¹ to 1200 cm⁻¹) were typically observed in silk fibroin. In particular, the amide I region, originating from the C=O stretching, suggests conformational changes. For silk fibroin, the β-sheet structure, which is in a crystalline form, is commonly indentified at 1620 cm⁻¹, whereas random coils and α-helix structures are generally found at 1640 cm⁻¹ to 1660 cm⁻¹.

Without ultrasonication, silk fibroin showed only a peak at 1638 cm⁻¹ (without a peak at 1620 cm⁻¹), thereby suggesting a non-crystalline structure. When ultrasonicated for an additional 5 seconds, there was a slight change without a β-sheet characteristic peak at 1620 cm⁻¹. However, the ultrasonication for 10 seconds or more showed a strong peak of a β-sheet structure. Another peak shift from 1510 cm⁻¹ to 1515 cm⁻¹ appeared in the amide II region, which is known to correspond to the secondary N-H bending and C-N stretching of a β-sheet. Such peak shift was also observed in all ultrasonicated samples, which is ascribed to the C-C ring stretching vibration of the tyrosine side chain, and this phenomenon in the amide II region is known to further adopt the conformation of the β-sheet structure.

Next, FT-IR analysis was performed for the collagen-silk composite hydrogels having different compositions. When compared with ultrasonicated pure silk, all of the collagen-silk composites showed a similar peak shift at around 1620 cm⁻¹ (amide I region), and the peak intensity was different depending on the composition, which was presumably due to the different collagen/silk contents. Such results show that the β-sheet formation of silk was not disturbed by the presence of collagen. In addition, close examination revealed that the peaks in the amide II region were variable depending on the collagen/silk compositions, *i.e*., different intensities and peaks incurred at 1510 cm⁻¹, 1515 cm⁻¹, or 1523 cm⁻¹, reflecting that there may be conformational changes in the β-sheet of silk due to the molecular interactions between the two components.

Similar molecular interactions between silk and collagen accelerate the gelation process, and consequently influence the physical and mechanical properties, as described below.

### Experimental Example 3: Dynamic mechanical analysis of hydrogels

Dynamic mechanical analysis of the hydrogels was performed. The viscoelastic properties of the collagen-silk hydrogels were investigated using an oscillatory shear rheometer. Groups in series I (CS-A to CS-H; Table 1) with a collagen/silk ratio fixed at 1:7 and variable concentrations were analyzed. For the test, 1 mL of each hydrogel solution was cased to a mold (diameter: 25 mm, height: 1.5 mm) and then gelated. The hydrogel solutions were covered with a thin layer of PDMS to flatten the surface. All measurements were carried out using an advanced rheometric expansion system (ARES LS2, TA instruments) having 25 mm of parallel plate geometry.

In order to determine the linear viscoelastic range (LVE), a strain sweep test was performed in a strain range of 0.01% to 100% at a frequency of 1 rad/s. The samples were then tested in a sweeping frequency range of 0.1 rad/s to 100 rad/s under 5% strain, which is within the LVE range. The shear moduli (storage G' and loss G") of hydrogels were recorded and the loss angle (tanθ = G"/G') was also calculated.

As a result of the measurement, the G' (storage modulus) and G" (loss modulus) values, which show the elastic component measuring the stored energy and the viscous portion from the energy lost as heat, respectively, were obtained. First, the G' and G" values of the hydrogels were recorded as a function of frequency, and then the changes in average G' value of the hydrogels were plotted in Fig. 3.

As the silk and collagen concentrations increased, the G' value increased almost exponentially, covering a wide range of stiffness levels, *i.e*., from 0.017 kPa (silk (0.35%) + collagen (0.5 mg/mL)) to 6.81 kPa (silk (2.45%) + collagen (3.5 mg/mL). In particular, the G' values of very soft hydrogels were: 0.017 kPa for 'CS-A', 0.079 kPa for 'CS-B', and 0.385 kPa for 'CS-D', all of which are considered to be relevant for the applications to very soft tissues, such as nerves.

Additionally, the G' values of medium-soft hydrogels (CS-C, CS-E, and CS-F) were 0.278 kPa, 1.19 kPa, and 1.53 kPa, and these were considered suitable for the applications to medium-soft tissues, such as muscles. On the other hand, the relatively stiff hydrogels recording G' values of 5.16 kPa (CS-G) and 6.8 kPa (CS-H) are useful for hard tissues, such as cartilage and bones. Accordingly, it was confirmed that the collagen-silk hydrogels of the present invention exhibited matrix stiffness that is appropriate for soft-to-hard tissues.

The storage modulus G' of most composite hydrogels (except 'CS-A') was significantly higher than the loss modulus G", and a loss factor (tanδ = G"/G') was much lower than 0.1 (Table 3). These results suggest that the composite hydrogels behave more like elastic solids rather than viscous liquids.

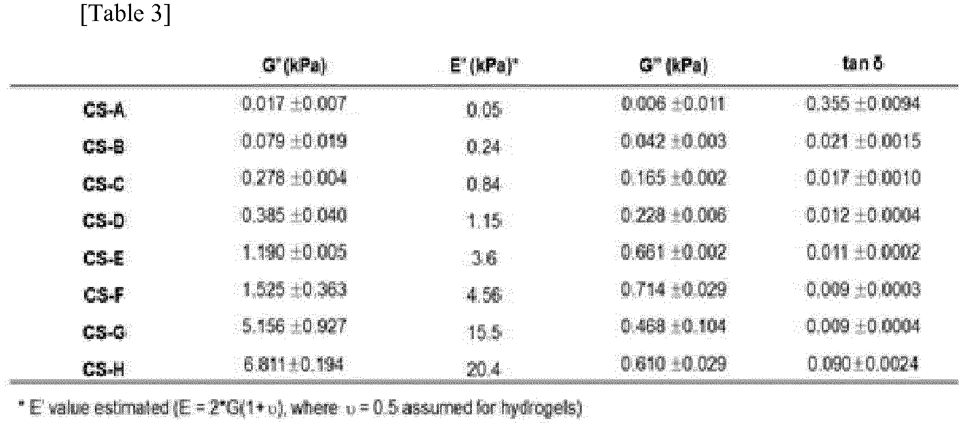

Elastic modulus (E') was estimated by assuming a simplified proportionality (Poisson ratio = 0.5).

Along with the experimental shear modulus values, the E' values were estimated by the following formula: E = 2·G (1 + υ), wherein υ is the Poisson ratio and assumed to be 0.5 for simplicity in the hydrogels.

As summarized in Table 3 above, the estimated E' values were in the range of 0.05 kPa to 20.4 kPa, and provided an approximated applicability of the collagen-silk hydrogels in direct comparison with the stiffness values of other hydrogels reported elsewhere. The fact that the stiffness can be adjusted along the iso-compositional line supports that the collagen-silk composite hydrogels of the present invention can be applied as matrix platforms showing stiffness against various cellular responses.

### Statistical analysis

Experiments were repeated three times, unless otherwise indicated. All different experiments were performed at least twice in order to confirm the reproducibility. Data were expressed as mean ± one standard deviation. Statistical analysis was performed by two-way analysis of variance, followed by a *post hoc* Bonferroni's correction (p < 0.05).

### Experimental Example 4: Mechanical responses of hydrogels

Mechanical responses of hydrogels under different stress modes were examined, together with the viscoelastic properties, by manually applying compression, bending, twisting, or elongation modes of stress, and by observing the shaping behaviors of hydrogels. Hydrogel samples with various shapes (*e.g.,* thin rectangular, tubular, and cylindrical shapes) were prepared, and then different stress modes (*e.g*., tension, shear/torsion, or compression) were manually applied. The collagen/silk composite (CS 1:7; 'CS-F' as a representative group) was compared with pure silk or collagen.

First, hydrogels with a thin rectangular shape were tested (Fig. 4a). 'CS-F' was selected as the control composite hydrogel group, and pure collagen (3.5 mg/mL) and pure silk (2%) were selected as the control hydrogel groups. Integrity, bending ability, elasticity, and water squeeze-out of hydrogels were observed using a flat rectangular shape. A solution (3 mL) was poured into a PDMS mold (3 cm × 3 cm × 0.3 mm) and incubated for 6 hours for complete gelation. The silk hydrogel preserved its shape when unmolded, but could not maintain the integrity due to a creamy property, and broke easily when held by a spatula. On the contrary, the collagen and collagen-silk hydrogels were easily bent when unmolded, exhibiting a characteristic elastic behavior. Additionally, the collagen hydrogel constantly lost water content, and its shape and mechanical integrity changed over time; however, such phenomenon was minimal in the collagen-silk hydrogel.

Next, hydrogels with a hollow tubular form were prepared and tested (Fig. 4b). The silk hydrogel exhibited a rather rigid behavior and was easily broken when bent or twisted. In addition, a dye solution injected through the hollow core was blocked and could not easily flow inside the channel. The collagen hydrogel, on the other hand, appeared to resist high stresses, such as torsion, bending, tension, *etc.,* but the shape change also accompanied plastic yield and water loss. In contrast, the composite hydrogel showed much higher elasticity against the applied stresses, maintaining its shape well against a high bending, twisting, or pulling stress, without water loss.

Hydrogels were prepared in a syringe (1 mL) with a mandrel (2 mm diameter) at the core. Resistance to a compression force of the hydrogels was observed using a cylindrical shape (10 mm diameter × 15 mm height).

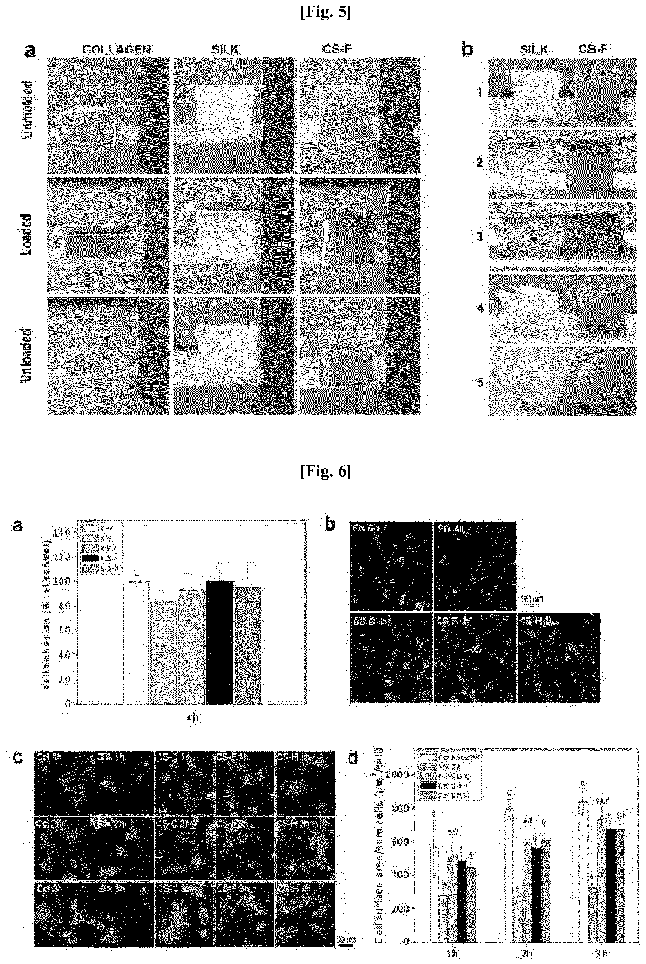

For the collagen hydrogel, when unmolded, even the initial shape could not be preserved due to a large loss of water (Fig. 5a). Once the water was sufficiently compressed, the shape of the collagen hydrogel was not significantly changed by one cyclic compression. However, the reported compressed collagen gel, in which water was squeezed out, was not elastic but rather stiff.

On the other hand, the silk hydrogel resisted a small cyclic load without water loss. Additionally, the composite hydrogel appeared to have low elastic deformation during the loading/unloading cycle. Although both silk and composite hydrogels appeared to behave similarly under a small cyclic load, these showed a clear difference under a high compressive load (Fig. 5b). A continuous compression with increasing stress led to failure of the silk hydrogel.

However, the composite hydrogel could resist high stresses, reflecting an excellent elastic property. As observed, the collagen-silk hydrogel showed intriguing mechanical behaviors. That is, the collagen-silk hydrogel was highly elastic, dynamically changing shape under various stress modes such as tension, bending, torsion/twisting, and compression, and resisting plastic failure; such feature is clearly different from that of pure collagen (easily loses water and yield) or that of pure silk (not elastic but rigid and difficult to mold). That is, the composite hydrogels of the present invention have an advantage over individual hydrogels in that they are applicable to numerous elastic tissues as compared with individual hydrogels.

### Experimental Example 5: Cellular responses of hydrogels

Cell anchorage and spreading behaviors were examined at 1 hour, 2 hours, and 3 hours. At each culture time, each sample was fixed with 4% paraformaldehyde for 20 minutes and then treated with 0.1% Triton X-100 for 7 minutes. After washing with PBS, the samples were blocked with a solution (3% bovine serum albumin in PBS) for 30 minutes.

Next, F-actin filaments and nuclei were stained by Alexa Fluor 546-conjugated phalloidin (Invitrogen, A22283) and DAPI (4',6-diamidino-2-phenylindole dihydrochloride) (Invitrogen, D1306), respectively. Cell images were obtained using an iRIS™ Digital Cell Image System (Logos Biosystems) and a confocal laser scanning microscope (Zeiss LSM 700). Cell spreading area and anchored cell number were calculated by means of ImageJ software from the 10 frames that were randomly selected for each group.

Human mesenchymal stem cells (hMSC; MSC-003, Rooster Bio, US) obtained from bone marrow were used for *in vitro* studies. Cells were scaled with a High Performance Media Kit (RoosterBio, KT-001) according to the manufacturer's instructions. Hydrogel samples for cell cultures were prepared with a disc shape (10 mm diameter × 200 µm height) using a PDMS mold.

CS-C, CS-F, CS-H, pure collagen (3.5 mg/mL), and pure silk (2%) were used. Before inoculating the cells, a drop of media (80 µL) was added to each hydrogel, incubated for 1 hour, and then removed. 1 × 10⁴ cells (60 µL) were seeded onto each hydrogel and allowed to attach.

Human mesenchymal stem cells (hMSC) were cultured on hydrogels and the anchorage and spreading behaviors were monitored for up to 4 hours (Fig. 6). Representative composite hydrogels in an iso-compositional line CS 1:7 with different stiffness levels (CS-C, CS-F, and CS-H) were used, and pure collagen and pure silk hydrogels were compared. After 4 hours, the hMSCs appeared to show almost complete saturation in cell attachment.

The counted number of the attached cells was slightly higher on pure collagen and collagen-silk composites (92% to 99% of collagen) than on pure silk (82% of collagen) (Fig. 6a). However, a closer look at the fluorescent cell morphology revealed that the cells on pure silk were round with limited spreading, whereas those on collagen and collagen-silk composites spread actively (Fig. 6b). There was no significant difference in the initial cell attachment level and shape among the composite hydrogel groups.

At earlier time points (1 hour, 2 hours, and 3 hours), the cell anchorage and spreading behaviors were significantly different (Figs. 6c and 6d). The hMSCs on the pure collagen hydrogel were highly stretched along with cytoskeletal extensions at as early as 1 hour, but the cells on the pure silk hydrogel were mostly round-shaped with almost no cytoskeletal processes up to 3 hours.

On the other hand, the cells on the collagen-silk composite hydrogel were initially round-shaped, but rapidly developed over time and exhibited extensive cytoskeletal processes. The cell spreading was quantified. The cells on pure collagen showed the widest spreading area at all time points, and those on pure silk showed the lowest spreading area. The collagen-silk composite hydrogel also allowed substantial cell spreading, with a significantly higher level than that of the pure silk hydrogel. It is known that silk from *B. mori* lacks cell adhesive ligands such as RGD sequences in its amino acid structure, and thus has limited initial cellular activity. Therefore, collagen mixed with the silk hydrogel could accelerate the hMSC adhesion and spread through adhesive ligands.

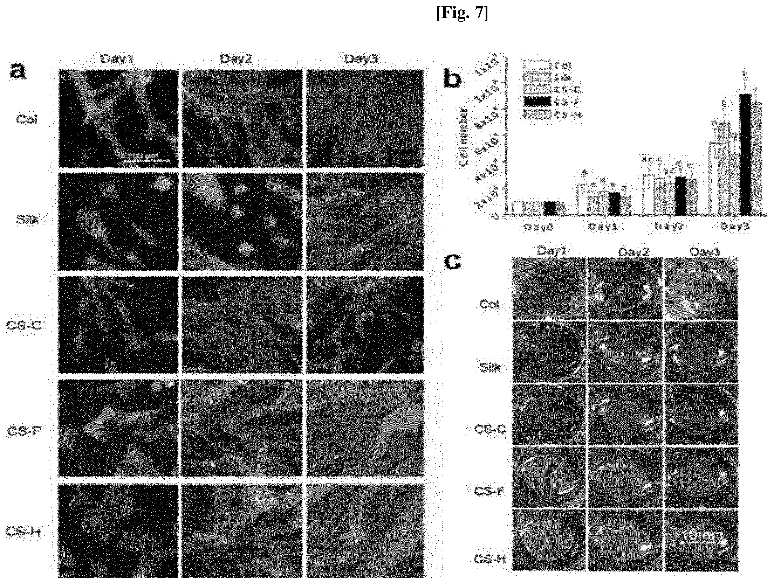

The hMSCs were tightly adhered and spread. Thereafter, these were divided over time for proliferation, and then examined by fluorescence microscopy during the culture for up to 3 days (Fig. 7a). The cells on the pure collagen hydrogel were highly elongated at day 1 such that cytoskeletal processes were developed along the collagen fibrils.

In prolonged cultures (2 days to 3 days), the cells actively proliferated to almost entirely cover the surface. On the pure silk hydrogel, at days 1 and 2, most of the cells were still round-shaped while some spread in a polygonal shape, and then at day 3, these became highly spread and proliferated to reach a confluence, similar to those observed on the collagen hydrogel. At day 3, the hMSCs were able to secrete ECM molecules including adhesive proteins that can significantly assist cell growth and spreading.

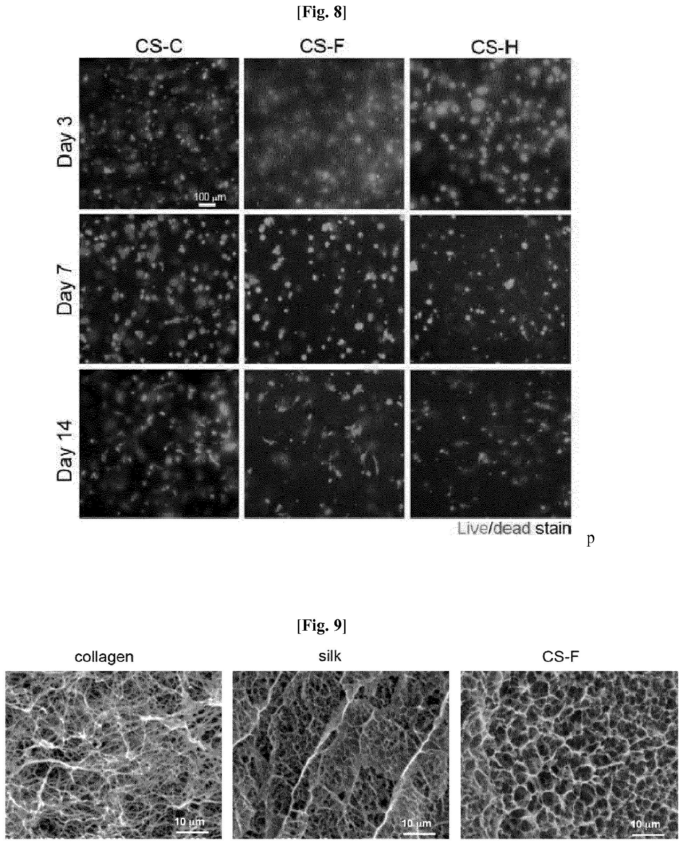

On the collagen-silk composite hydrogel, the cells actively spread similarly to those on the pure collagen hydrogel, but the shape was polygonal (different from the elongated shape in pure collagen), reflecting the underlying matrix microstructure. That is, the collagen-silk network did not have a collagenous fiber morphology, but exhibited a general random porous morphology (similar to the pure silk network mentioned in Fig. 9), in which collagen molecules are spatially distributed to assist initial cell spreading and proliferation.

With prolonged cultures of 2 days to 3 days, the cells on the composite hydrogels behaved differently depending on the groups. That is, the cells on CS-F and CS-H continued to spread and actively proliferate, almost completely covering the surface, similar to the cells on the pure collagen hydrogel, whereas the cells on CS-C appeared to shrink in their proliferative potential from day 2 to day 3.

The number of cells measured during the cultures from day 1 to day 3 exhibited significant differences among the groups; CS-F and CS-H enabled active proliferation of cells over time, but CS-F did not (Fig. 7b). The densely networked hydrogels (CS-F and CS-H), due to their relatively high stiffness, allowed hMSCs to preserve active growth and all-directional spreading with a polygonal shape from day 2 to day 3. However, the cytoskeletal processes of the cells on the loosely networked hydrogel (CS-C), due to its soft property, could maintain the polygonal shape at day 2, and could reconstitute the network structure after changing to a more elongated shape. At day 3, about 90% of the hydrogel had shrunk, and the number of the cells had increased 3-fold from the cells at day 1 (Fig. 7c). In fact, this phenomenon has been commonly observed in the cell cultures with collagen gels, and is also considered a significant concern for many biomedical uses. The traction forces of cells exerted on the collagen fibers could be prevalent while cell population increased, and as a result, this led to a significant shrinkage of gels.

Unlike pure collagen, the pure silk hydrogel did not exhibit such shrinkage during the culture up to 3 days. In addition, all collagen-silk hydrogel groups preserved their initial shape without shrinkage. If the cell proliferation levels of pure collagen and composite hydrogels were similar at day 3, the cell exerting forces might be comparable. Therefore, the higher resistance to gel shrinkage of the composite hydrogels was considered to be attributed to the improved mechanical properties of the composite networks. That is, due to the elastic property of the silk-collagen networks, the composite hydrogels of the present invention respond more dynamically to cells compared to pure collagen, thereby resisting yield (a phenomenon occurring in pure collagen networks) against cellular traction forces.

### Experimental Example 6: Cell encapsulation within hydrogels

After confirming that the composite hydrogels of the present invention provided excellent matrix conditions for 2D culturing of hMSCs, which includes cell anchorage, spreading, and proliferation, cells were cultured in 3D conditions by encapsulating them during the hydrogel preparation. For cell encapsulation, 5 × 10⁵ cells (100 µL) were added to hydrogels (1 mL), as described in the literature (X. Wang, J.A. et al., Biomaterials. 29 (2008) 10541064). The cells were always added to a collagen solution after neutralization at pH 7.0 before mixing with the ultrasonicated silk solution. The cell-embedded hydrogel (35 µL) was added to a PDMS mold and cultured for 2 hours, and the medium was added thereto, and then cultured for another period of time.

The encapsulated cells were able to survive and grow, but due to high silk concentrations, the stiffness level was very high (63 kPa to 441 kPa), and as a result, these are limited to use in the engineering of hard tissues such as bones. However, in the present invention, much lower silk concentrations (0.35% to 2.45%) could easily be manipulated into cell-encapsulating hydrogels by means of combining with collagen; as a result, the stiffness shifted towards a much softer range (0.017 kPa to 6.81 kPa), and thus cell-encapsulating hydrogels suitable for hard tissues, *e.g*., brain and muscle, could be produced.

hMSCs were encapsulated in three composite hydrogels (CS-C, CS-F, and CS-H, respectively), which are in the iso-compositional line (CS 1:7) but have different silk-collagen concentrations, and then cell viability was observed during the culture on a growth medium for 14 days. As shown in the live/dead cell stain images (Fig. 8), it was confirmed that most cells remained alive over the long-term cultures in all composite hydrogels.

## Claims

1. A method for preparing a composite hydrogel for cell encapsulation comprising collagen and silk fibroin, which comprises forming a gel by mixing collagen having an α-helix structure and silk fibroin having a β-sheet structure.

2. The method according to claim 1, wherein the collagen is prepared by neutralizing the collagen to be self-assembled into collagen having an α-helix structure.

3. The method according to claim 1 or 2, wherein the silk fibroin is prepared by ultrasonicating the silk fibroin to be self-assembled into silk fibroin having a β-sheet structure.

4. The method according to any one of claims 1 to 3, comprising forming a gel by mixing the collagen having an α-helix structure (0.5 mg/mL to 4 mg/mL) and the silk fibroin having a β-sheet structure (0.35% w/v to 2.45% w/v); and forming an interpenetrating network by molecular interaction.

5. The method according to any one of claims 1 to 4, wherein the stiffness of the hydrogel is adjusted within the range of 0.05 kPa to 40 kPa by adjusting the collagen and silk fibroin concentrations.

6. The method according to any one of claims 1 to 5, wherein the collagen before the neutralization has a triple helix structure.

7. The method according to any one of claims 1 to 6, wherein the ultrasonication is conducted from 10 seconds to 60 seconds at 20% amplitude.

8. The method according to any one of claims 1 to 7, wherein rigidity is adjusted by adjusting the collagen concentration and the silk fibroin concentration along an iso-compositional line.

9. The method according to any one of claims 1 to 8, wherein the molecular interaction is a hydrogen bond.

10. A composite hydrogel for cell encapsulation comprising collagen and silk fibroin, wherein:
the composite hydrogel comprises collagen having an α-helix structure and silk fibroin having a β-sheet structure, in which an interpenetrating network between the collagen having an α-helix structure and the collagen having a β-sheet structure is formed by a hydrogen bond; and
the stiffness of the composite hydrogel is adjusted within the range of 0.05 kPa to 40 kPa.
